(19) Europäisches Patentamt
European Patent Office
Office européen des brevets

(11) Publication number: **0 035 255**
**B1**

(12) # EUROPEAN PATENT SPECIFICATION

(45) Date of publication of patent specification: **30.05.84**

(51) Int. Cl.$^3$: **C 07 H 21/00,** C 07 H 19/04

(21) Application number: **81101444.8**

(22) Date of filing: **27.02.81**

(54) **Process for removing trityl blocking groups from 5'-O trityl nucleosides and oligonucleotides.**

(30) Priority: **29.02.80 US 126025**

(43) Date of publication of application:
**09.09.81 Bulletin 81/36**

(45) Publication of the grant of the patent:
**30.05.84 Bulletin 84/22**

(84) Designated Contracting States:
**AT BE CH DE FR GB IT LI NL**

(56) References cited:

**Carbohydrate Research Vol 65(1978) pages 132-138**

**TETRAHEDRON LETTERS, vol. 21, pages 719-722 Pergamon Press M.D. MATTEUCCI et al.: "The synthesis of oligodeoxypyrimidines on a polymer support"**

**TETRAHEDRON LETTERS 1976, pages 809-812 Pergamon Press E.J. COREY et al.: "A new general method for protection of the hydroxyl function"**

(73) Proprietor: **UNIVERSITY PATENTS, INC.**
**537 Newtown Avenue**
**Norwalk Connecticut 06851 (US)**

(72) Inventor: **Caruthers, Marvin H.**
**1450 Kendall Drive**
**Boulder, Colorado (US)**
Inventor: **Matteucci, Mark D.**
**3490 Berkely**
**Boulder, Colorado (US)**

(74) Representative: **Brauns, Hans-Adolf, Dr. rer. nat. et al**
**Hoffmann. Eitle & Partner, Patentanwälte**
**Arabellastrasse 4**
**D-8000 Munich 81 (DE)**

(56) References cited:
**JUSTUS LIEBIGS: "Annalen der Chemie", 1978, Heft 6, pages 839-853 Verlag Chemie, Weinheim, DE H. KOSTER et al.: "Synthese eines Strukturgens für das Peptidhormon Angiotensin II, Teil 1, Zielsetzung und Synthesestrategie**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 035 255 B1**

(56) References cited:
**CHEMICAL REVIEWS, 1977, vol. 77, no. 2, pages 183-217 V. AMARNATH et al.: "Chemical Synthesis of Oligonucleotides"**

**TETRAHEDRON LETTERS, vol. 21, pages 3243-3246, 1980 Pergamon Press M.D. MATTEUCCI et al.: "The use of zinc bromide for removal of dimethoxytrityl ethers from deoxynucleosides"**

## Description

The present invention relates to a new and useful process for removing blocking groups from nucleosides and oligonucleotides and is especially useful in synthetic processes for producing oligonucleotides and polynucleotides.

In the chemical synthesis of oligonucleotides and polynucleotides, there is the need to block the 5′ —O— position of the nucleosides to assure proper condensation reactions to occur at other positions in the nucleoside molecule, specifically at the 3′ —O— position as is well known in the art. In this regard, see J.A.C.S. *97*, 3278—3279 (1975); *ibid*, *98*, 3655—3661 (1976); and Proc. Int. Conf. Transfer RNA, Poznan, Poland, October 1976 pp. 145—155. The most commonly employed blocking groups are trityl groups, especially methoxy substituted trityl groups such as p-anisyldiphenylmethyl and di-p-anisylphenylmethyl. Such groups can be removed with some ease by use of protic acids but protic acids can have a deleterious effect on the nucleoside base, particularly where the base is a purine, resulting in depurination of the nucleoside, even where only stoichiometric amounts of protic acid are employed. Of course, the effect of the protic acid is to reduce the yield of desired product.

In Carbohydrate Research, 65 (1978), pages 132—138 a simple method for detritilation of carbohydrates is described. According to this method trityl groups can be cleaved rapidly and without side reactions by using boron trifluoride to yield the corresponding compounds with deprotected primary hydroxyl groups.

In Tetrahedron Letters (1976) No. 11, pages 809—812 a general method for the protection of the hydroxyl function is described. The protecting group disclosed is a B-methoxymethyl (MEM) group which is suitable for protection of primary, secondary and tertiary alcohols. Said protecting group is removable under aprotic conditions under the influence of a mild Lewis acid. There is no suggestion that the method disclosed in the above methods would be operable or even desirable with respect to nucleosides or oligonucleotides which are not simple carbohydrate derivatives, but either purine or pyrimidine derivatives.

The present invention provides a new and useful process for removing the trityl blocking groups from the 5′ —O— position of a nucleoside. The process is accomplished by contacting the blocked nucleoside molecule with a Lewis acid and the blocking group is removed. The reaction occurs at room temperature which is of course preferred although higher or lower temperatures can be used. Since only short reaction times of only a few minutes are required at room temperature, the use of higher, or even lower, temperatures is not indicated. However, temperatures as low as even 0°C and up to reflux temperature of the reaction mixture can be used.

The contacting of blocked nucleoside with Lewis acid is usually effected in a reaction solvent. A wide variety of solvents can be used including for example acetonitrile, nitromethane, dioxane, tetrahydrofuran and mixtures thereof. These solvents of course can be diluted with other solvents such as acetone, methylene chloride and the like.

The solvents are preferably dry in order to avoid hydrolysis of those Lewis acids which are water-sensitive. Such hydrolysis would lead to protic acid production which can cause depurination, for example, and is preferably avoided by using essentially water-free solvents.

Any Lewis acid can be employed in the present process but the halide salts of metals such as aluminum, zinc, titanium and boron are preferred. In particular, zinc bromide is an especially effective Lewis acid in the present process, although aluminum chloride, boron trifluoride and titanium tetrachloride also give excellent results.

The use of Lewis acids in the hydrolysis of 5′ —O— trityl nucleosides, deoxynucleosides, oligonucleotides, oligodeoxynucleotides, polynucleotides and polydeoxynucleotides is superior to the use of protic acids since the reaction is extremely fast and avoids the deleterious effect, e.g., depurination, caused by the use of protic acids. As is apparent from comparative data based on identical runs using a Lewis acid versus a protic acid, the time required for significant depurination with Lewis acids, e.g. zinc bromide, is quite long and the removal of the trityl group occurs within relatively short periods, at room temperature and even at 0°C. In contrast, significant depurination occurs with protic acids in short time periods and this accounts for the significant depurination which accompanies use of protic acids to remove 5′ —O— trityl groups.

A further advantage of the use of Lewis acids in the present process is the specificity for the 5′-O-trityl group of the nucleoside. Thus, when the nucleoside includes trityl blocking groups in both the 3′ —O— and 5′ —O— positions, it is possible to remove only the 5′ —O— trityl with the Lewis acid in accordance with the present invention. Accordingly, it is possible to make 3′ —O— tritylnucleosides using the process of the present invention on 3′,5′-ditritylnucleosides as starting material. The specificity of the present process for the 5′-trityl group is thus of substantial value in providing implementation of oligonucleotide synthesis starting with 3′-trityl blocked nucleosides and oligonucleotides.

The nucleosides and oligonucleotides used in the present invention includes a variety thereof. Thus, the simple, unmodified nucleosides or oligonucleotides used in chemical synthesis of polynucleotide can be employed as described in the hereinbeforementioned literature references. Additionally, such nucleosides and oligonucleotides can be attached on to organic polymers as described in *Chemical Reviews, 77*, 183—217 (1977).

Further, the nucleoside and oligonucleotides can be attached to inorganic polymers such as silica gel through base hydrolyzable covalent linkages as described in copending US-application Ser. No. 126,025 filed February 29, 1980 (priority document of the present application), wherein modified silica gel is produced by the steps of: treating silica gel particles with an organo-silane to provide a reaction product of silica gel having organo-silane side chains covalently bonded thereto; treating said reaction product with means to provide terminal carboxylic acid functionality to at least some of the side chains; and treating with material to block free silicic acid functionality on said silica gel, and nucleosides are then linked to the modified silica gel at the carboxyl acid function.

In the synthesis of oligonucleotides and polynucleotides, the blocking of the 5′ —O— position is essential to assure condensation reactions through the 3′ —O— position through which phosphorus functionality is introduced to form internucleotide linkages as depicted by the following:

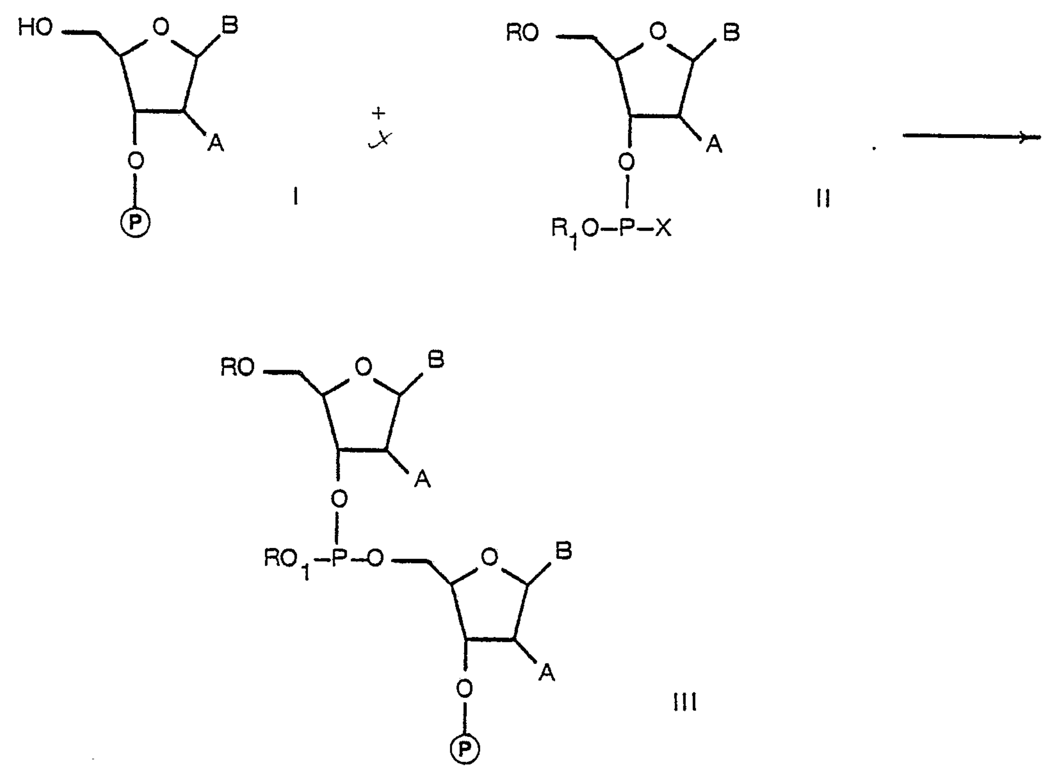

I

II

III

wherein Ⓟ is an inorganic polymer linked to the 3′ or 5′ —O— of the nucleoside through a base hydrolyzable covalent bond; R is H or a blocking group; $R_1$ is a hydrocarbyl radical containing up to 10 carbons; X is halogen, preferably Cl or Br or a secondary amino group attached through the amino nitrogen; each B is nucleoside or deoxynucleoside base; and each A is H or OR.

As is apparent the condensation requires blocking of the 5′ —O— position in the phosphinylating compound II and removal of the 5′-trityl group from the product III is required to permit increasing the nucleoside chain. As is set out in claims 10—13, trityl blocking groups are removed from di-p-anisyl phenyl methyl or p-anisyl diphenyl methyl nucleosides or oligonucleotides covalently bound to an inorganic polymer support by contacting the said nucleosides or oligonucleotides with zinc bromide under essentially anhydrous conditions.

The following examples further illustrate the invention.

Example

The procedure employed involves contacting the 5′-tritylnucleoside with zinc bromide in the reaction solvent, nitromethane.

The rate of detritylation was measured and comparative data is provided in Table I.

TABLE I

The Rate of Detritylation and Depurination of 5′-Dimethoxytrityl-N-benzoyl-deoxyadenosine Using Various Solutions*

| Solution | Temp. | Detritylation Time | % | Depurination Time | % |
|---|---|---|---|---|---|
| satd. $ZnBr_2/CH_3NO_2$** | 18°C | <1 min. | 100 | 10 h. | 50 |
| satd. $ZnBr_2/CH_3NO_2$ | 0°C | 10 min. | 100 | 21 h. | <5 |
| 2% toluenesulfonic acid/$CHCl_3$:$CH_3OH$ (7:3) | 18°C | <1 min. | 100 | 5 min. | 50 |
| 0.5% toluenesulfonic acid/$CHCl_3$:$CH_3OH$ (7:3) | 0°C | 10 min. | 100 | 8 h. | 50 |

*All depurination results summarized in this communication were obtained by analyzing reaction mixtures using reverse phase high pressure liquid chromatography. Estimates of detritylation times were by thin layer chromatography.
**A nitromethane solution saturated with $ZnBr_2$ is approximately 0.1 M in $ZnBr_2$.

The results obtained with various tritylthymidines at room temperature with zinc bromide is given in Table II.

TABLE II

The rate of Detritylation and Degradation of 5′-Trityldeoxynucleosides Using $ZnBr_2$ at Room Temperature. <1 min.

| Deoxynucleoside | Detritylation Time | % | Degradation Time |
|---|---|---|---|
| 5′-Dimethoxytrityl-N-isobutyryl deoxyguanosine | <1 min. | 100 | 50 h. |
| 5′-Dimethoxytrityl-N-benzoyldeoxycytosine | <1 min. | 100 | 24 h. |
| 5′-Dimethoxytritylthymidine | <1 min. | 100 | 24 h. |

and at 0°C in Table III.

TABLE III

The Rate of Detritylation of Various Tritylthymidines Using Saturated $ZnBr_2$ in Nitromethane at 0°C.

| Nucleoside | Time | % Detritylation |
|---|---|---|
| 5′-Dimethoxytritylthymidine | 1 min. | 50 |
| 5′-Monomethoxytritylthymidine | 1 min. | 50 |
| 5′-Tritylthymidine | 10 min. | 50 |
| 3′-Monomethoxytritylthymidine | 30 min. | 10 |

The detritylation procedure is not restricted to polymer support synthesis but is also useful for strictly solution synthesis procedures where the use of trityl groups is included in the reaction sequence.

**Claims**

1. A process for removing trityl blocking groups from 5′ —O— trityl nucleosides and oligonucleotides which comprises contacting said nucleoside or oligonucleotide with an aprotic Lewis acid under essentially anhydrous conditions.

2. The process according to Claim 1 wherein the nucleoside or oligonucleotide is attached to an inorganic support through a covalent linkage.

3. The process according to Claim 1 wherein the nucleoside or oligonucleotide is attached to a silica gel through a base — hydrolyzable covalent linkage.

4. The process according to Claim 1 wherein the Lewis acid is zinc bromide, aluminum chloride, boron trifluoride or titanium tetrachloride.

5. The process according to Claim 1 wherein contacting is effected in the presence of nitromethane as reaction solvent.

6. The process according to Claim 1 wherein said trityl group is di-p-anisylphenylmethyl or p-anisyldiphenylmethyl.

7. A process for removing trityl blocking groups from 5′ —O— trityl nucleosides or oligonucleotides which comprises contacting said nucleoside or oligonucleotide with zinc bromide under essentially anhydrous conditions.

8. The process according to Claim 7 wherein said trityl group is di-p-anisylphenylmethyl or p-anisyldiphenylmethyl.

9. The process according to Claim 7 wherein contacting is effected in the presence of nitromethane as reaction solvent.

10. The process according to Claim 7 wherein the nucleoside or oligonucleotide is attached to a silica gel through a base — hydrolyzable covalent linkage.

11. A process for removing trityl blocking groups from di-p-anisylphenylmethyl or p-anisyldiphenylmethylnucleosides or oligonucleotides covalently bound to an inorganic polymer support which comprises contacting the said nucleosides or oligonucleotides with zinc bromide under essentially anhydrous conditions.

12. The process according to Claim 11 wherein said inorganic polymer support is silica gel covalently bound to said nucleoside or oligonucleotide through an ester linkage.

13. The process according to Claim 11 wherein contacting is effected in the presence of nitromethane as reaction solvent.

**Patentansprüche**

1. Verfahren zum Entfernen von Trityl-Blockierungsgruppen aus 5′ —O— Tritylnucleosiden und -Oligonucleotiden, dadurch gekennzeichnet, dass man das Nucleosid oder Oligonucleotid mit einer aprotischen Lewis-Säure unter im wesentlichen wasserfreien Bedingungen in Berührung bringt.

2. Verfahren gemäss Anspruch 1, dadurch gekennzeichnet, dass das Nucleosid oder Oligonucleotid an einen anorganischen Träger mittels kovalenter Bindungen angehaftet ist.

3. Verfahren gemäss Anspruch 1, dadurch gekennzeichnet, dass das Nucleosid oder Oligonucleotid an ein Kieselgel durch basenhydrolysierbare kovalente Bindungen angehaftet ist.

4. Verfahren gemäss Anspruch 1, dadurch gekennzeichnet, dass die Lewis-Säure Zinkbromid, Aluminiumchlorid, Bortrifluorid oder Titantetrachlorid ist.

5. Verfahren gemäss Anspruch 1, dadurch gekennzeichnet, dass man den Kontakt in Gegenwart von Nitromethan als Reaktionslösungsmittel vornimmt.

6. Verfahren gemäss Anspruch 1, dadurch gekennzeichnet, dass die Tritylgruppe Di-p-anisylphenylmethyl oder p-Anisyldiphenylmethyl ist.

7. Verfahren zum Entfernen von Trityl-Blockierungsgruppen aus 5′ —O— Tritylnucleosiden oder -Oligonucleotiden, dadurch gekennzeichnet, dass man das Nucleosid oder Oligonucleotid mit Zinkbromid unter im wesentlichen wasserfreien Bedingungen behandelt.

8. Verfahren gemäss Anspruch 7, dadurch gekennzeichnet, dass die Tritylgruppe Di-p-anisylphenylmethyl oder p-Anisyldiphenylmethyl ist.

9. Verfahren gemäss Anspruch 7, dadurch gekennzeichnet, dass man die Kontaktierung in Gegenwart von Nitromethan als Reaktionslösungsmittel vornimmt.

10. Verfahren gemäss Anspruch 7, dadurch gekennzeichnet, dass das Nucleosid oder Oligonucleotid mittels einer basenhydrolysierbaren kovalenten Bindung an ein Kieselgel angehaftet ist.

11. Verfahren zum Entfernen von Trityl-Blockierungsgruppen aus Di-p-anisylphenylmethyl- oder p-Anisyldiphenylmethylnucleosiden oder -oligonucleotiden, welche kovalent an einen anorganischen polymeren Träger gebunden sind, dadurch gekennzeichnet, dass man die Nucleoside oder Oligonucleotide mit Zinkbromid unter im wesentlichen wasserfreien Bedingungen in Kontakt bringt.

12. Verfahren gemäss Anspruch 11, dadurch gekennzeichnet, dass der anorganische polymere Träger ein Kieselgel ist, welcher mittels einer Esterbindung kovalent an das Nucleosid oder Oligonucleotid gebunden ist.

13. Verfahren gemäss Anspruch 11, dadurch gekennzeichnet, dass man das Kontaktieren in Gegenwart von Nitromethan als Reaktionslösungsmittel vornimmt.

**Revendications**

1. Procédé pour enlever les groupes protecteurs trityle de 5′ —O— trityl nucléosides et oligonucléotides caractérisé en ce qu'il consiste à mettre en contact un tel nucléoside ou oligonucléotide avec un acide de Lewis aprotique dans des conditions essentiellement anhydres.

2. Procédé selon la revendication 1 caractérisé en ce que le nucléoside ou oligonucléotide est attaché à un support inorganique au moyen d'une liaison covalente.

3. Procédé selon la revendication 1 caractérisé en ce que le nucléoside ou oligonucléotide est attaché à un gel de silice au moyen d'une liaison covalente d'une base hydrolysable.

4. Procédé selon la revendication 1 caractérisé en ce que l'acide de Lewis est un des suivants: bromure de zinc, chlorure d'aluminium, trifluorure de bore, tétrachlorure de titane.

5. Procédé selon la revendication 1 caractérisé en ce que la mise en contact est effectuée en présence de nitrométhane servant de solvant de réaction.

6. Procédé selon la revendication 1 caractérisé en ce que le groupe trityle est di-p-anisylphénylméthyle ou p-anisyldiphénylméthyle.

7. Procédé pour enlever les groupes protecteurs trityle de 5'—O— trityl nucléosides ou oligonucléotides caractérisé en ce qu'il consiste à mettre en contact un tel nucléoside ou oligonucléotide avec du bromure de zinc dans des conditions essentiellement anhydres.

8. Procédé selon la revendication 7 caractérisé en ce que le groupe trityle est di-p-anisylphénylméthyle ou p-anisyldiphénylméthyle.

9. Procédé selon la revendication 7 caractérisé en ce que la mise en contact est effectuée en présence de nitrométhane servant de solvant de réaction.

10. Procédé selon la revendication 7 caractérisé en ce que le nucléoside ou oligonucléotide est attaché à un gel de silice au moyen d'une liaison covalente d'une base hydrolysable.

11. Procédé pour enlever les groupes protecteurs trityle de di-p-anisylphénylméthyl- ou p-anisyldiphénylméthyl-nucléosides ou oligonucléotides liés de manière covalente à un support polymère inorganique caractérisé en ce qu'il consiste à mettre en contact ces nucléosides ou oligonucléotides avec du bromure de zinc dans des conditions essentiellement anhydres.

12. Procédé selon la revendication 11 caractérisé en ce que le support polymère inorganique est du gel de silice lié de manière covalente au nucléoside ou oligonucléotide au moyen d'un liaison ester.

13. Procédé selon la revendication 11 caractérisé en ce que la mise en contact est effectuée en présence de nitrométhane servant de solvant de réaction.